# EUROPÄISCHE PATENTSCHRIFT

(11) **EP 0 598 269 B1**
(45) Veröffentlichungstag und Bekanntmachung des Hinweises auf die Patenterteilung: **17.03.1999**
(21) Anmeldenummer: 93117660.6
(22) Anmeldetag: 02.11.1993
(51) Int. Cl.: C12M 3/00, C12M 1/00

(54) **Vorrichtung zum Kultivieren von lebenden Zellen und Geweben**
Apparatus for the culture of living cells and tissues
Appareil pour la culture de cellules et tissus vivants

(30) Priorität: 17.11.1992 DE 4238722
(43) Veröffentlichungstag der Anmeldung: 25.05.1994
(73) Patentinhaber: KENDRO Laboratory Products GmbH, 63450 Hanau (DE)
(72) Erfinder: Fenner, Manfred, D-63828 Kleinkahl (DE); Heeg, Hubert, D-63776 Mömbris (DE)
(74) Vertreter: Kühn, Hans-Christian

(56) Entgegenhaltungen:
- DE-A- 2 924 446
- US-A- 3 117 009

## Beschreibung

Die Erfindung betrifft eine Vorrichtung zum Kultivieren von lebenden Zellen und Geweben mit einem Nutzraum, dessen Atmosphäre über eine Luftbefeuchtungseinrichtung befeuchtbar ist, die einen Flüssigkeitsvorratsbehälter aufweist, der über eine Durchführung in einem Verdampfer mit einem im Nutzraum angeordneten Dampfaustrittsteil durch Leitungen flüssigkeitsmäßig verbunden ist, wobei der Eintritt der Leitung in das Dampfaustrittsteil auf einem höheren Niveau angeordnet ist als das Flüssigkeitsniveau des Verdampfers und mit einer Kondenswasser aus dem Nutzraum beführenden Ablaufleitung.

Eine derartige Vorrichtung ist aus der DE 29 24 446 bekannt. Die hier beschriebene Vorrichtung weist einen in ihrem unteren Teil angeordneten Flüssigkeitsvorratsbehälter auf, von dem die Flüssigkeit mittels einer Pumpe in den Verdampfer transportiert wird. Von dort wird die verdampfte Flüssigkeit in den Nutzraum geleitet. Unterhalb des Dampfaustritts ist in dem Nutzraum eine Kühlfalle angeordnet, unterhalb der sich eine Kondenswasserauffangschale befindet. Von dort wird das Kondenswasser über einen Auffangbehälter von einer Pumpe durch den Verdampfer in die Umgebung abgeführt. Diese Vorrichtung ist relativ aufwendig, da zum einen das Kondensat im Nutzraum separat unter Einsatz einer Kühlfalle gesammelt wird und zum anderen zwei Pumpen für den Flüssigkeitstransport benötigt werden. Da die Pumpen füllstandsabhängig geschaltet werden, müssen an mehreren Stellen der Vorrichtung Füllstandsmesser angeordnet sein.

Ausgehend von diesem Stand der Technik liegt der vorliegenden Erfindung die Aufgabe zugrunde, eine Vorrichtung der eingangs beschriebenen Art, mit einem einfachen und wartungsarmen mechanischen Aufbau bereitzustellen.

Diese Aufgabe wird dadurch gelöst, daß der Flüssigkeitsvorratsbehälter sowohl auf einem höheren Niveau als das Flüssigkeitsniveau des Verdampfers und auch als der Eintritt der Leitung in das Dampfaustrittsteil angeordnet ist, wobei das Dampfaustrittsteil als Kamin ausgebildet ist, in dessen oberen Bereich der Eintritt für die Leitung mündet und in dessen Bodenbereich die Ablaufleitung für das Kondenswasser ansetzt. Dabei fließt die Flüssigkeit für die Luftbefeuchtung im Nutzraum allein aufgrund der Schwerkraft in den Verdampfer, wird dort verdampft und sterilisiert. Der Dampf wird dem Dampfaustrittsteil im Inneren des Nutzraums zugeführt. Durch die spezielle Form des Dampfaustrittsteils als Kamin, aus welchem der Dampf in den Nutzraum eintritt, gelangt das eventuell sich bildende Kondenswasser nicht in den Nutzraum, sondern sammelt sich im Bodenbereich des Kamins und wird von dort aus dem Nutzraum abgeführt. Bei einer solchen Anordnung sind keinerlei Pumpen oder andere bewegliche Teile für den Flüssigkeitstransport notwendig. Auch die aus dem Stand der Technik bekannte zusätzliche Kühlfalle kann entfallen, da der Bodenbereich des Kamins aufgrund seiner Verbindung nach außen selbst als Kühlfalle wirkt. Ein derartiger Aufbau ist damit nahezu wartungsfrei und sehr kostengünstig. Die Betankung des Flüssigkeitsvorratsbehälter erfolgt üblicherweise über einen Einfüllstutzen.

Zweckmäßigerweise ist in der Leitung zwischen Flüssigkeitsvorratsbehälter und Verdampfer ein Magnetventil angeordnet, das über eine Steuerschaltung mit einem im Nutzraum angeordneten Feuchtemesser verbunden ist. Über dieses Magnetventil läßt sich der Zufluß der Flüssigkeit aus dem Flüssigkeitsvorratsbehälter und damit die dem Nutzraum zugeführte Dampfmenge exakt steuern. Dadurch ist in Verbindung mit dem im Nutzraum angeordneten Feuchtemesser eine genaue Regelung der Luftfeuchtigkeit im Nutzraum möglich. Durch das Magnetventil wird weiterhin eine Quantelung der dem Verdampfer zugeführten Flüssigkeitsmenge erreicht, so daß gesichert werden kann, daß die dem Verdampfer zugeführte Flüssigkeit nahezu vollständig verdampft.

Zweckmäßigerweise ist das dem Flüssigkeitsvorratsbehälter zugewandte Ende der Durchführung durch den Verdampfer auf gleichem oder niedrigerem Niveau angeordnet als das dem Dampfaustrittsteil zugewandte Ende. Durch diese Anordnung wird ebenfalls gesichert, daß die dem Verdampfer zugeführte Flüssigkeit nahezu vollständig verdampft, da ein zu schnelles Durchströmen des Verdampfers aufgrund der Schwerkraftwirkung verhindert wird.

Um den Verdampfer möglichst einfach zu gestalten, ist es vorteilhaft, daß die Durchführung die Form einer gegenüber der waagerechten geneigten Geraden aufweist. Insbesondere ist es vorteilhaft, daß die Durchführung um einen Winkel von etwa 20 bis 30° gegenüber der Waagerechten geneigt ist; jedoch sind auch andere Neigungen, bis hin zu 90°, denkbar. Durch die Neigung wird insbesondere verhindert, daß nichtverdampftes Wasser durch den Verdampfer hindurchtritt.

Zweckmäßigerweise weist der Verdampfer einen beheizbaren massiven Metallkörper auf. Dieser Metallkörper bewirkt, daß kurzfristige Temperaturschwankungen innerhalb des Verdampfers vermieden werden und damit bei einer hohen Temperaturkonstanz eine gleichmäßige Verdampfung der Flüssigkeit erfolgt. Dazu ist es insbesondere vorteilhaft, daß die Durchführung durch den Metallkörper hindurch geführt ist.

Zweckmäßigerweise wird die Ablaufleitung ebenfalls durch den Verdampfer geführt, so daß das Kondenswasser verdampft und dann steril der Umgebungsluft zugeführt wird. Dazu ist es vorteilhaft, daß die Ablaufleitung in ein Verdunstungsgefäß mündet. Zweckmäßigerweise ist dieses Verdunstungsgefäß derart gestaltet, daß die Ablaufleitung in den oberen Bereich des Verdunstungsgefäßes mündet und das Verdunstungsgefäß einen Kondensatablauf aufweist, der zwischen Dampfaustrittsteil und Verdampfer mit der Ablaufleitung verbunden ist. Dadurch wird die vollständige Verdunstung des Kondenswassers gewährleistet, da auch das in dem Verdunstungsgefäß anfallende Kondenswasser erneut verdampft wird.

Zweckmäßigerweise ist der Kondensatablauf auf einem niedrigeren Niveau angeordnet als der Bodenbereich des Dampfaustrittsteils. Durch diese Anordnung wird sichergestellt, daß zuerst das Kondenswasser aus dem Dampfaustrittsteil dem Verdampfer zugeführt wird und nicht etwa das unter Umständen nicht keimfreie Kondenswasser aus dem Verdunstungsgefäß außerhalb des Nutzraumes in das Dampfaustrittsteil im Inneren des Nutzraums hineingedrückt wird. Auf diese Weise ist auch gesichert, daß Kondenswasser aus dem Dampfaustrittsteil stets abgeführt wird, so daß keine unkontrollierte Veränderung der Luftfeuchtigkeit im Inneren des Nutzraumes dadurch eintreten kann, daß bei geschlossenem Magnetventil eventuell im Dampfaustrittsteil befindliches Kondenswasser in dem Nutzraum verdunstet.

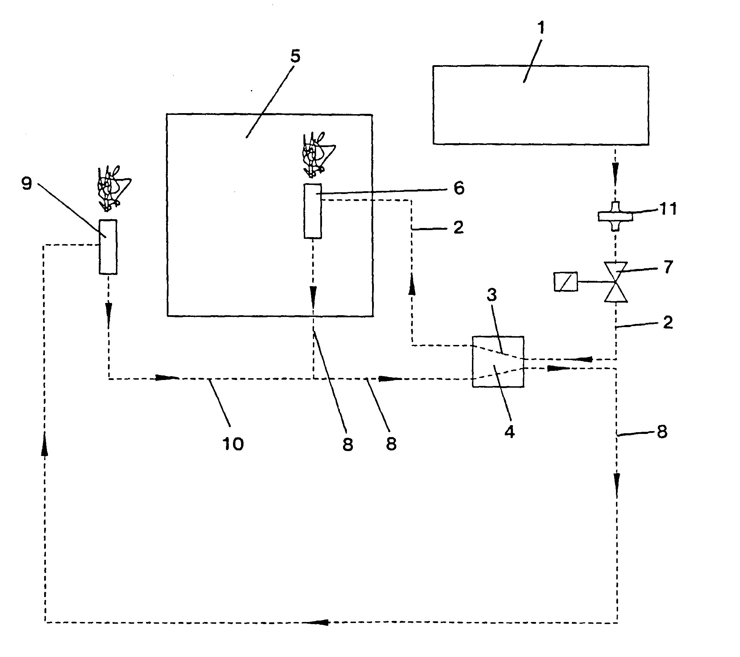

Nachfolgend wird die Erfindung anhand eines Ausführungsbeispieles und einer Zeichnung näher erläutert. Die Zeichnung zeigt die schematische Darstellung der Vorrichtung.

Im oberen Bereich der Vorrichtung befindet sich der Flüssigkeitsvorratsbehälter 1. Der Boden des Flüssigkeitsvorratsbehälters 1 befindet sich auf einem höheren Niveau als das Flüssigkeitsniveau in der Durchführung 3 des Verdampfers 4 und als das im Nutzraum 5 angeordnete Dampfaustrittsteil 6, wobei der Eintritt der Leitung 2 in das Dampfaustrittsteil 6 auf einem höheren Niveau angeordnet ist als das Flüssigkeitsniveau in der Durchführung 3.

Im Boden des Flüssigkeitsvorratsbehälters 1, der das destillierte Wasser für die Luftbefeuchtung des Nutzraumes 5 enthält, ist die Leitung 2 angeschlossen. Die Leitung 2 ist über Durchführung 3 in dem Verdampfer 4 mit dem Dampfaustrittsteil 6 verbunden. Durch diese Anordnung wird das destillierte Wasser allein durch Schwerkraftverteilung in den Verdampfer 4 transportiert. In der Leitung 2 befindet sich zwischen dem Flüssigkeitsvorratsbehälter 1 und dem Verdampfer 4 ein Magnetventil 7, das über eine nicht dargestellte Steuerschaltung mit einem im Nutzraum 5 angeordneten, ebenfalls nicht dargestellten Feuchtemesser, verbunden ist. Dem Magnetventil 7 ist ein Filter 11 vorgelagert, um eine Verunreinigung des Magnetventils 7 zu verhindern. Das Magnetventil 7 arbeitet im Taktbetrieb (beispielsweise 30 ms ein, 1 s aus). Dadurch wird das dem Verdampfer 4 zugeführte Wasser portioniert. Insgesamt werden dem Verdampfer 4 auf diese Weise etwa 2 ml/min zugeführt. Der Verdampfer 4, der im wesentlichen aus einem elektrisch beheizten Metallblock besteht, weist eine Temperatur von etwa 500°C auf. Bei dieser Temperatur verdampft das zugeführte Wasser und gleichzeitig wird der Wasserdampf bei der hohen Temperatur sterilisiert, so daß keimfreier Dampf in das Dampfaustrittsteil 6 und damit in den Nutzraum 5 gelangt. Die Menge des in dem Verdampfer 4 erzeugten Dampfes läßt sich durch Neigen der Durchführung 3 durch den Verdampfer 4 variieren. So beträgt die Kondensatmenge, d. h. der Anteil an mitgerissenem Wasser bei waagerechter Anordnung der Durchführung 3 etwa 50 %, bei einer Neigung um etwa 15° gegenüber der Waagerechten, wobei das dem Dampfaustrittsteil 6 zugewandte Ende der Durchführung 3 höher liegt, nur noch etwa 30 %. Diese Werte sind jedoch auch abhängig von der Länge der Durchführung 3, von der Menge des zugeführten Wassers, von dem Höhenunterschied zwischen Flüssigkeitsvorratsbehälter 1 und der Durchführung 3, von dem Durchmesser der Durchführung 3 usw.

Da das Dampfaustrittsteil 6 als Kamin ausgebildet ist, in dessen oberen Bereich die Leitung 2 für die Zuleitung des Dampfes mündet, gelangt das mitgerissene bzw. im Dampfaustrittsteil 6 gebildete Kondenswasser in dessen Bodenbereich, in dem die Ablaufleitung 8 ansetzt. Der Bodenbereich des Dampfaustrittsteils 6 ist oberhalb des Flüssigkeitsniveaus der Ablaufleitung 8 in dem Verdampfer 4 angeordnet. Dadurch wird das Kondenswasser der Schwerkraft folgend dem Verdampfer 4 zugeführt und dort erneut verdampft. Der Dampf wird einem ebenfalls außerhalb des Nutzraumes 5 gelegenen Verdunstungsgefäß 9 zugeführt. Dieses Verdunstungsgefäß 9 ist wie das Dampfaustrittsteil 6 in Form eines Kamins gestaltet, in dessen oberen Bereich die Ablaufleitung 8 mündet und in dessen Boden der Kondensatablauf 10 ansetzt. Der Kondensatablauf 10 ist zwischen dem Dampfaustrittsteil 6 und dem Verdampfer 4 in die Ablaufleitung 8 eingekoppelt, so daß auch das aus dem Verdunstungsgefäß 9 abgeführte Kondenswasser dem Verdampfer 4 erneut zugeführt wird. Dadurch wird lediglich keimfreier Dampf an die Umgebung abgegeben.

Der Boden des Verdunstungsgefäßes 9 ist auf einem niedrigeren Niveau angeordnet als der Bodenbereich des Dampfaustrittsteiles 6, um zu verhindern, daß Kondenswasser aus dem Verdunstungsgefäß 9 in das Dampfaustrittsteil 6 gedrückt wird und hier zu einer unkontrollierten Verdunstung und damit zu einer unkontrollierten Luftfeuchtigkeit im Nutzraum 5 führen kann. Auf diese Weise ist gewährleistet, daß das im Dampfaustrittsteil 6 gebildete Kondenswasser stets sofort abläuft und umgekehrt ein Einlaufen des aus dem Verdunstungsgefäß kommenden und eventuell nicht sterilen Kondenswassers in das Dampfaustrittsteil 6 verhindert wird.

## Patentansprüche

1. Vorrichtung zum Kultivieren von lebenden Zellen und Geweben mit einem Nutzraum, dessen Atmosphäre über eine Luftbefeuchtungseinrichtung befeuchtbar ist, die einen Flüssigkeitsvorratsbehälter aufweist, der über eine Durchführung in einem Verdampfer mit einem im Nutzraum angeordneten Dampfaustrittsteil durch Leitungen flüssigkeitsmäßig verbunden ist, wobei der Eintritt der Leitung in das Dampfaustrittsteil auf einem höheren Niveau angeordnet ist als das Flüssigkeitsniveau des Verdampfers und mit einer Kondenswasser aus dem Nutzraum abführenden Ablaufleitung, dadurch gekennzeichnet, daß der Flüssigkeitsvorratsbehälter (1) sowohl auf einem höheren Niveau als das Flüssigkeitsniveau des Verdampfers (4) und auch als der Eintritt der Leitung (2) in das Dampfaustrittsteil (6) angeordnet ist, wobei das Dampfaustrittsteil (6) als Kamin ausgebildet ist, in dessen oberen Bereich der Eintritt für die Leitung (2) mündet und in dessen Bodenbereich die Ablaufleitung (8) für das Kondenswasser ansetzt.

2. Vorrichtung nach Anspruch 1, dadurch gekennzeichnet, daß in der Leitung (2) zwischen Flüssigkeitsvorratsbehälter (1) und Verdampfer (4) ein Magnetventil (7) angeordnet ist, das über eine Steuerschaltung mit einem im Nutzraum angeordneten Feuchtemesser verbunden ist.

3. Vorrichtung nach Anspruch 1 oder 2, dadurch gekennzeichnet, daß das dem Flüssigkeitsvorratsbehälter (1) zugewandte Ende der Durchführung (3) durch den Verdampfer (4) auf gleichem oder niedrigerem Niveau angeordnet ist als das dem Dampfaustrittsteil (6) zugewandte Ende.

4. Vorrichtung nach Anspruch 3, dadurch gekennzeichnet, daß die Durchführung (3) die Form einer gegenüber der Waagerechten geneigten Geraden aufweist.

5. Vorrichtung nach Anspruch 4, dadurch gekennzeichnet, daß die Durchführung (3) um einen Winkel von etwa 20 bis 30° gegenüber der Waagerechten geneigt ist.

6. Vorrichtung nach einem der Ansprüche 1 bis 5, dadurch gekennzeichnet, daß der Verdampfer (4) einen beheizbaren massiven Metallkörper aufweist.

7. Vorrichtung nach Anspruch 6, dadurch gekennzeichnet, daß die Durchführung (3) durch den Metallkörper geführt ist.

8. Vorrichtung nach einem der Ansprüche 1 bis 7, dadurch gekennzeichnet, daß die Ablaufleitung (8) durch den Verdampfer (4) geführt ist.

9. Vorrichtung nach Anspruch 8, dadurch gekennzeichnet, daß die Ablaufleitung (8) in ein Verdunstungsgefäß (9) mündet.

10. Vorrichtung nach Anspruch 9, dadurch gekennzeichnet, daß die Ablaufleitung (8) in den oberen Bereich des Verdunstungsgefäßes (9) mündet und das Verdunstungsgefäß (9) einen Kondensatablauf (10) aufweist, der zwischen Dampfaustrittsteil (6) und Verdampfer (4) mit der Ablaufleitung (8) verbunden ist.

11. Vorrichtung nach Anspruch 10, dadurch gekennzeichnet, daß der Kondensatablauf (10) auf einem niedrigeren Niveau angeordnet ist als der Bodenbereich des Dampfaustrittsteils (6).

## Claims

1. Apparatus for cultivating living cells and tissues, having a working space whose atmosphere can be moistened by an air humidifying means which has a liquid storage container which is connected by liquid-carrying lines via a passage in an evaporator to a vapour outlet part arranged in the working space, the entrance of the line into the vapour outlet part being arranged at a higher level than the liquid level of the evaporator, and having a drain line which removes the condensation water from the working space, characterized in than the liquid storage container (1) is arranged at a higher level than both the liquid level of the evaporator (4) and the entrance of the line (2) into the vapour outlet part (6), the vapour outlet part (6) being in the form of a chimney into the upper region of which the inlet for the line (2) opens and in the bottom region of which the drain line (8) for the condensation water starts.

2. Apparatus according to Claim 1, characterized in than a solenoid valve (7) is arranged in the line (2) between liquid storage container (1) and evaporator (4) and is connected via a control circuit to a humidity meter arranged in the working space.

3. Apparatus according to Claim 1 or 2, characterized in that that end of the passage (3) through the evaporator (4) which faces the liquid storage container (1) is arranged at the same level as or a lower level than the end facing the vapour outlet part (6).

4. Apparatus according to Claim 3, characterized in that the passage (3) has a shape of a straight line inclined relative to the horizontal.

5. Apparatus according to Claim 4, characterized in that the passage (3) is inclined at an angle of about 20 to 30° relative to the horizontal.

6. Apparatus according to any of Claims 1 to 5, characterized in that the evaporator (4) has a heatable solid metal body.

7. Apparatus according to Claim 6, characterized in that the passage (3) passes through the metal body.

8. Apparatus according to any of Claims 1 to 7, characterized in that the drain line (8) passes through the evaporator (4).

9. Apparatus according to Claim 8, characterized in than the drain line (8) opens into an evaporation vessel (9).

10. Apparatus according to Claim 9, characterized in than the drain line (8) opens into the upper region of the evaporation vessel (9) and the evaporation vessel (9) has a condensate drain (10) which is connected to the drain line (8), between the vapour outlet part (6) and the evaporator (4).

11. Apparatus according to Claim 10, characterized in that the condensate drain (10) is arranged at a lower level than the bottom region of the vapour outlet part (6).

## Revendications

1. Appareil pour la culture de cellules et de tissus vivants, comportant un compartiment utile dont l'atmosphère peut être humidifiée à l'aide d'un dispositif d'humidification de l'air, qui présente un réservoir de liquide relié par des conduites de liquide, par l'intermédiaire d'un passage dans un évaporateur, à un élément d'échappement de vapeur disposé dans le compartiment utile, l'entrée de la conduite dans l'élément d'échappement de vapeur étant située à un niveau supérieur au niveau du liquide de l'évaporateur, et à une conduite d'évacuation faisant sortir l'eau de condensation hors du compartiment utile, caractérisé en ce que le réservoir de liquide (1) est disposé à un niveau supérieur aussi bien au niveau de liquide de l'évaporateur (4) qu'à l'entrée de la conduite (2) dans l'élément d'échappement de vapeur (6), l'élément d'échappement de vapeur (6) ayant une forme de cheminée dans la zone supérieure de laquelle débouche l'entrée de la conduite (2) et au fond de laquelle est raccordée la conduite d'évacuation (8) pour l'eau de condensation.

2. Appareil selon la revendication 1, caractérisé en ce qu'une vanne magnétique (7) est disposée dans la conduite entre le réservoir de liquide (1) et l'évaporateur (4), et est reliée par l'intermédiaire d'un circuit de commande à un dispositif de mesure de l'humidité placé dans le compartiment utile.

3. Appareil selon la revendication 1 ou 2, caractérisé en ce que l'extrémité du passage (3) dans l'évaporateur (4) se trouvant du côté du réservoir de liquide (1) est disposée au même niveau ou à un niveau plus bas que l'extrémité se trouvant du côté de l'élément d'échappement de vapeur (6).

4. Appareil selon la revendication 3, caractérisé en ce que le passage (3) a la forme d'une droite inclinée par rapport à l'horizontale.

5. Appareil selon la revendication 4, caractérisé en ce que le passage (3) forme un angle d'inclinaison d'environ 20 à 30° par rapport à l'horizontale.

6. Appareil selon l'une des revendications 1 à 5, caractérisé en ce que l'évaporateur (4) comporte un corps métallique massif pouvant être chauffé.

7. Appareil selon la revendication 6, caractérisé en ce que le passage (3) passe à travers le corps métallique.

8. Appareil selon l'une des revendications 1 à 7, caractérisé en ce que la conduite d'évacuation (8) passe dans l'évaporateur (4).

9. Appareil selon la revendication 8, caractérisé en ce que la conduite d'évacuation (8) débouche dans un récipient de vaporisation (9).

10. Appareil selon la revendication 9, caractérisé en ce que la conduite d'évacuation (8) débouche dans la partie supérieure du récipient de vaporisation (9) et en ce que le récipient de vaporisation (9) comporte une conduite d'évacuation de l'eau condensée (10) qui est reliée à la conduite d'évacuation (8) entre l'élément d'échappement de vapeur (6) et l'évaporateur (4).

11. Appareil selon la revendication 10, caractérisé en ce que la conduite d'évacuation de l'eau condensée (10) est disposée à un niveau inférieur au fond de l'élément d'échappement de vapeur (6).
